# EUROPEAN PATENT APPLICATION

(11) **EP 2 962 627 A1**
(43) Date of publication of application: **06.01.2016**
(21) Application number: 13876641.5
(22) Date of filing: 28.02.2013
(51) Int. Cl.: A61B 3/10

(54) **SYSTEM FOR OBTAINING PARAMETERS FOR ADJUSTING FRAMES WITH LENSES FOR A USER**

(71) Applicant: Tecnología Pro Informática, S.L., 46020 Valencia (ES); Asociación Industrial de Óptica, Color e Imagen - AIDO, 46980 Paterna (ES)
(72) Inventor: ROS SIMÓ,Rafael A., E-46020 Valencia (ES); PASCUAL FORTES, José Miguel, E-46980 Paterna (ES); ESTEVE TABOADA, José Juan, E-46980 Paterna (ES); MICÓ SERRANO, Vicente, E-46980 Paterna (ES); HERVÁS JUAN, Juan, E-46980 Paterna (ES); SIMÓN MARTÍN, Santiago, E-46980 Paterna (ES)
(74) Representative: Carvajal y Urquijo, Isabel
(86) International application number: PCT/ES2013/070125
(87) International publication number: WO 2014/131917

(57) **Abstract**

The invention relates to a system for producing parameters for adjusting frames with lenses, including a stereoscopic image generation module (70) for simultaneously generating an image composed of two simultaneous sub-images of a user with different perspectives. The system also includes a camera (8) for recording the stereoscopic image generated by the stereoscopic image generation module (70). The system also has means (60) for processing information from the recorded images, that are used to estimate different adjustment parameters. Furthermore, the system can include a lighting module (10) that is optically associated with the stereoscopic image generation module (70) and designed to produce a bright stimulus in the optical infinity of the user, that allows relaxed accommodation and convergence.

## Description

### OBJECT OF THE INVENTION

The object of the present invention is an automatic optical system for obtaining parameters for adjusting frames with lenses, a solution applicable to the field of optics - optometry, which is based on the use of a stereoscopic image capture system, image processing means and user interaction means, all that in such a way that the required parameters are obtained in a fast and simple way.

### BACKGROUND OF THE INVENTION

Document US2009/0021693 A1 from the prior art refers to a device for determining the optical parameters of a user and it discloses at least two image recording devices to respectively generate the image of sub-sections of the user head. For an appropriate operation this device requires taking two images, each one coming from a different camera. Regarding the way of capturing the image, both images feature a time delay one with respect to the other. This is achieved making a beam splitter change from completely transparent to completely reflective from one image to the other. Furthermore, the pupil center is determined from the corneal reflex of the lamps placed over the cornea. This may imply a carry-over error because of the effect that the corneal curvature may introduce in both perspectives.

### DESCRIPTION OF THE INVENTION

The present invention solves the limitations identified in the state of the art. Since they are generated at the same instant of time, it is possible to achieve no differences in the patient conditions between the two perspectives being captured. Also, thanks to the stereoscopic image generation module, only one camera is required. It is also possible to obtain a stereoscopic measurement of the interpupillary distance, that is, it is possible to obtain the pupils coordinates in the three-dimensional space (3D), thereby providing a higher accuracy than that obtained by manually measuring with a ruler. This fact also allows the measurement of the interpupillary distance to be tolerant to the patients head rotations.

The stereoscopic measurement is obtained from two perspectives (one of them preferably in a front view) in a single image, captured with a single camera and an optical system designed for this purpose.

Another differentiating factor with respect to traditional systems is that detection of the pupil centers can be performed automatically. The system disclosed preferably uses a high-intensity lighting module which makes it possible to guarantee the appropriate contrast in the captured image so as to be able to perform the automatic detection of the pupil centers. It is the pupil centers themselves that are detected, and not the corneal reflections of the lighting module, as it happens in other existing systems. This is achieved by applying techniques of digital image processing.

Likewise, by using stereoscopy it becomes unnecessary to use any device coupled to the frame in order to establish a reference that makes it possible to obtain the measurement parameters.

Preferably, the stereoscopic image generation module is included within the system head block, as it is described below in an exemplary embodiment.

Advantageously, another additional characteristic of the system is that the measurement of the interpupillary distance can be taken under far vision conditions. To this end, a stimulus placed in the optical infinity is used as a fixing point, by means of an optical module especially designed for this purpose.

Additionally, the system may include a reflection mechanism which makes it possible for the patient to automatically locate in the best measurement conditions instantly, making the system much easier to use.

And finally, the system optionally enables direct interaction for communicating and exchanging data with the management commercial tools, such as for example Visual GESOPT. A bidirectional communication is established, such that when the process starts the management sends the identification data of the optician and the patient, and once the process ends the system sends back the parameters measured for the patient together with the image obtained from the patient with the frame, in such a way that these data can be represented on a full-scale in the management display.

The parameters sent back to the management may be: nasal-pupillary distances, mounting heights according to boxing system, the frame box size, frame bridge size, lens diameters, height of the progressive corridor, pantoscopic and Galbe angle, distance to the vertex, data of the image scale and locations of the pupils and optical centers of the lenses.

Another advantage of the system is that it requires minimum maintenance, since all the elements are conveniently fixed to avoid undesired movements and the lighting and image capturing active modules do not require regular maintenance. The reason why an active lighting module is included is that, since the optician's shops are highly illuminated it is very habitual to find strong reflections in the demo lenses when capturing the image. The active lighting module minimizes the effect of these reflections, turning the system immune to the different lighting conditions of the place in which it is located.

The final results can be provided in a report format having full-scale images, in such a way that the optician-optometrist can directly check the measurements using the physical frame placed on the printed report.

To summarize, the system may comprise the following functionalities, among others:
- Obtaining all adjusting parameters required to adapt the frames with lenses, being said parameters:
   - IPD: interpupillary distance.
   - NPD: Nasal-pupillary distance.
   - Lower height: distances between the pupil center of each eye and the base-frame.
   - Diameters of the lenses on the frame chosen by the patient.
   - Evaluating the frame measurements (for pre-gauging) according to the boxing system which defines the distances between pupils and the rectangles enclosing the two inner rings of the frame.
      ▪ Width and height of the Box.
      ▪ Upper and lower distance from the optical center to the upper and lower edges of the box.
      ▪ Nasal and temporal distance from the optical center to the nasal and temporal edges of the box.
   - Facial angle or Galbe angle forming the glasses dihedrals.
   - Pantoscopic angle forming the side profile of the frame with the vertical.
   - Vertex distance measuring the space between the pupil and the frame lens.
- Obtaining precise 3D measures by means of technology based on stereoscopic vision. A single capture and an optical system designed for this purpose allow the generation of a single image (stereoscopic image) from two different perspectives.
- Stereoscopic measurement, thus obtaining more precision than with the manual ruler. Obtaining real 3D coordinates of the pupils, being robust with respect to the patient's head rotations.
- Automatic detection of the pupil centers, not based on corneal reflexes.
- High-intensity lighting system which makes it possible to guarantee the required contrast and to eliminate undesired reflections on the lenses.
- Measurement taken under far vision conditions.
- Reflection system which allows an automatic and quick positioning by the patient.
- Personalization and simplification of the adjusting and adapting process of the lenses.
- Very simple to use, and quick in obtaining results automatically.

### BRIEF DESCRIPCTION OF THE FIGURES

The following is a brief description of each one of the drawings which will help understand the invention better and which clearly relates to an embodiment of said invention which is presented as a non-limiting example thereof.
FIGURE 1 shows an optical scheme of the stereoscopic module included within the head block of the system.
FIGURE 2 schematically shows an image taken by the system camera made up of two sub-images with a different perspective.
FIGURE 3 is a perspective view of the system assembly incorporated in an apparatus.
FIGURE 4 is a schematic drawing of the optical system included within the head block which makes it possible to show a virtual image of the light stimulus in the optical infinity of the user so that they fix their sight under far vision conditions.

### EMBODIMENT OF THE INVENTION

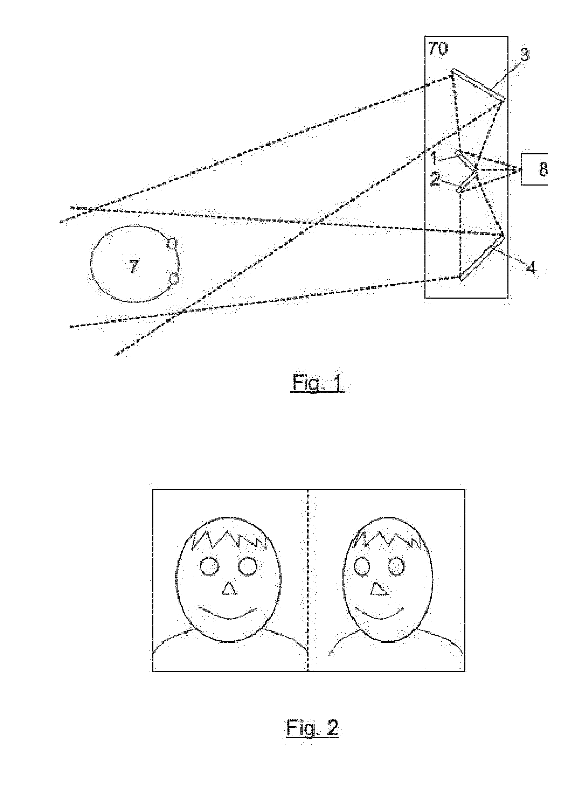

The optical scheme proposed in the present invention is that shown in FIGURE 1. The elements 1, 2 and 3 are totally reflective first surface mirrors. Element 4 is a beam splitter that reflects a percentage of the incident light and that transmits the rest. With this optical system, the stereoscopic image captured by the camera 8 is made up of two sub-images: the sub-image on the right is formed by the image of the patient's head 7 provided by mirrors **1** and **3.** The sub-image on the left is formed by the image of the patient's head provided by mirrors **2** and **4.**

FIGURE 2 schematically shows how the image captured by the system camera looks like, where it can be observed that two different perspectives of the patient's head are simultaneously captured in the same image.

Detection of the pupil centers can be performed automatically. To this end, the images obtained in the capture process are treated using image processing techniques for the analysis and extraction of the characteristics searched for. The image processing techniques being used are based on classical algorithms of noise reduction, edge enhancement, contrast increment and searching for defined patterns.

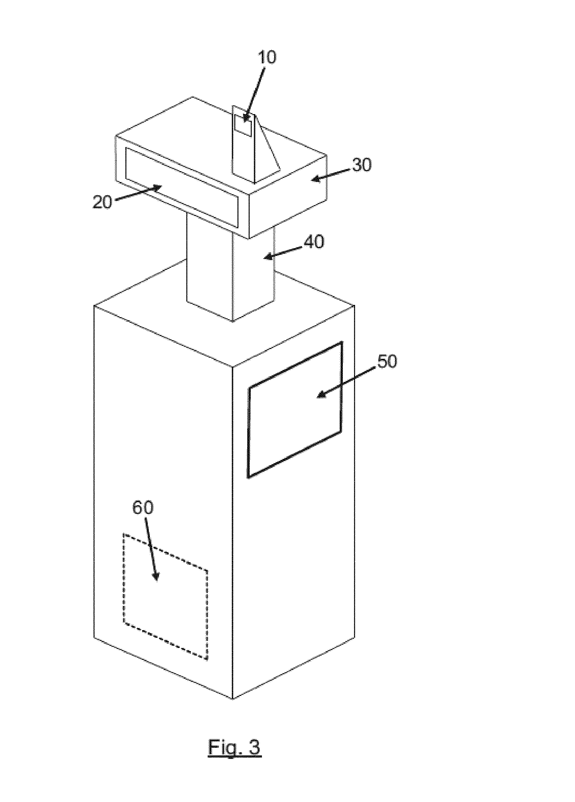

FIGURE 3 shows an exemplary embodiment according to the invention. A high-intensity lighting module **10** makes it possible to guarantee the appropriate contrast in the image captured, so as to be able to automatically detect the pupil centers themselves instead of the corneal reflexes, as it happens in other existing systems.

However, the manual option is available for the user to locate the pupil centers in the desired position. Automatic detection of the pupil centers is carried out with no need of using any special supports arranged on the frame. Furthermore, the high-intensity lighting module **10** allows the automatic detection of the pupil centers even in the case in which the lenses or the demo lenses (pieces of plastic that simulate lenses) have been removed.

The high-intensity lighting system **10** is located on a head block **30,** vertically aligned with the elements **4** and **5,** and having the appropriate tilt to evenly illuminate the patient face. This lighting module is activated at the instant the image is captured by the **8.** Thus, the system becomes immune to reflections that occur on the lenses of the user glasses.

The system may include a light stimulus in the optical infinity (approximately 6 meters), so that the user sight can be relaxed when focusing. Therefore, images are captured under far vision conditions (relaxed accommodation and convergence). This fact becomes important since when the stimuli are nearer to the optical infinity, the measurements are not obtained under far vision conditions, and therefore, the visual axes of the patient may be not parallel but convergent. This means that the area of the glasses the user is looking through is not the appropriate one since it does not correspond to the far vision area, which is the area where the lenses should be centered on.

In order to include a light stimulus in the optical infinity without modifying the optical system, the present embodiment includes, as it is shown in FIGURE 1, a beam splitter **4** that allows a percentage of the incident light to pass through and reflects the rest of the incident light.

Additionally, through the protection element **20** located in the head block **30,** the user can check the right location with respect to the system by means of their own reflection.

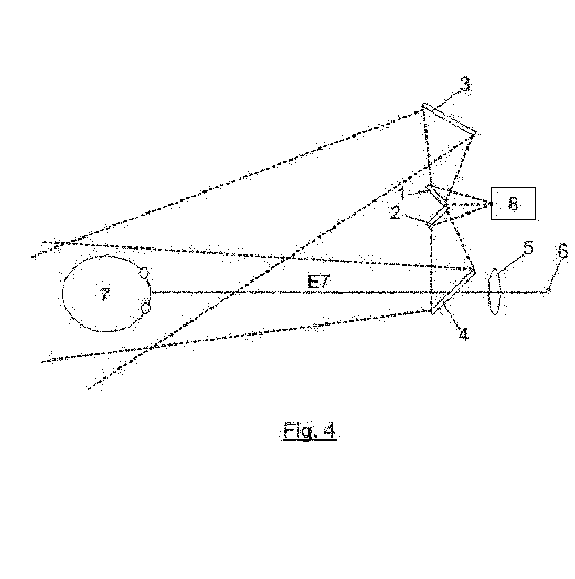

FIGURE 4 shows an optical system that includes the light stimulus in the optical infinity. The light stimulus is situated behind the beam splitter **4,** and the user is always aligned with its axis **E7** forming the lens **5** and the beam splitter **4** itself, and on which said user is located being aligned.

The light stimulus in the optical infinity is provided by a lens **5** and a led **6.** The lens **5** forms a virtual image of the led **6** in the optical infinity. The user is aligned with the system by means of the axis **E7,** forming the virtual image of the led **6.** In turn, the axis **E7** coincides with the central axis of the left sub-image of the camera, provided by elements **2** and **4.** For this reason, in the image captured by camera **8** in FIGURE 2, the patient always appear in the front view in the left sub-image (looking at the led image **6** through axis **E7**), while always appearing in a side perspective in the right sub-image (formed by first surface mirrors **1** and **3**).

To make it easier for the patient to be aligned comfortably and quickly with axis **E7,** the system includes a reflection element which can be an almost transparent protection surface **20** (slightly mirrored) that covers partially the head block **30** at its front. Thus, the patient can be located under the best measuring conditions in a simple manner, which makes the system operation easier and improves the step of obtaining the measurements.

### Number references

1, 2, 3 first surface mirrors;
4 beam splitter;
5 lens;
6 lighting source;
7 patient's head;
8 high resolution camera;
10 lighting module;
20 slightly mirrored almost transparent surface;
30 head block;
40 elevating means;
50 visualizing means;
60 processing means;
70 stereoscopic image generation module;
E7: axis of the lens 5 and the splitter 4.

## Claims

1. System for obtaining parameters for adjusting frames with lenses for a user, **characterized in that** it comprises:
- a stereoscopic image generation module (70) configured to generate, at the same instant, a stereoscopic image featuring two simultaneous sub-images with different perspectives of the user;
- a camera (8) configured to take the stereoscopic image;
- processing means (60) to process the information from the stereoscopic image taken by camera (8), with said processing means (60) being configured to estimate at least one of the following adjusting parameters:
Interpupillary distance, nasal-pupillary distances, distances between the pupil centers of each eye and the base-frame, lens diameters on the frame, width and height of a rectangle enclosing the pupils and the inner rings of the frame, nasal and temporal distance, Galbe angle, pantoscopic angle, distance to the vertex.

2. System according to claim 1, **characterized in that** a sub-image generated by the stereoscopic image generation module (70) is a front perspective.

3. System according to claim 1 or 2, **characterized in that** it also comprises a lighting module (10) optically associated with the stereoscopic image generation module (70) configured to produce a light stimulus in the optical infinity of the user.

4. System according to claim 3, **characterized in that** the lighting module (10) comprises a light source (6) and a lens (5) to produce a virtual image of said light source (6).

5. System according to any of the previous claims, **characterized in that** it also comprises a module assisting the user positioning (20) configured to validate the user position.

6. System according to claim 4 and 5, **characterized in that** it comprises a slightly mirrored almost transparent surface, designed to generate a reflection of at least a portion of the user face when they are correctly placed.

7. System according to any of the previous claims, **characterized in that** the stereoscopic image generation module (70) comprises three first surface mirrors (1,2,3) being totally reflective and at least one beam splitter (4).

8. System according to claims 3 and 7, **characterized in that** the beam splitter (4) is optically aligned with the light source (6).

9. System according to claim 7 or 8, **characterized in that** the light source (6), the lens (5) and the beam splitter (4) form an optical axis aligned with the user face when they are correctly placed.

10. System according to any of the previous claims, **characterized in that** it comprises a head block (30) to accommodate the camera (8) and the stereoscopic image generation module (70).

11. System according to claim 10, **characterized in that** it comprises means for the elevation (40) of the head block (30);

12. System according to any of the previous claims, **characterized in that** it comprises visualization means (50) configured to feature at least one of the adjusting parameters obtained and/or the image taken.

13. System according to any of the previous claims, **characterized in that** it comprises printing means configured to print a report having full-scale images of a portion of the user's face.

14. System according to claim 13, **characterized in that** the visualization means (50) comprise an interface configured to control the operation of at least one of the following elements:
- the stereoscopic image generation module (70),
- the camera (8),
- the processing means (60),
- the lighting module (10),
- the user positioning assistance module (20),
- the printing means.

15. System according to claim 14, **characterized in that** the visualization means (50) comprise a touch screen.
